# EUROPÄISCHE PATENTANMELDUNG

(11) **EP 3 483 351 A2**
(43) Veröffentlichungstag der Anmeldung: **15.05.2019**
(21) Anmeldenummer: 18213129.2
(22) Anmeldetag: 18.12.2015
(51) Int. Cl.: E03D 9/05, A61L 9/12, E03D 9/052, E03D 9/00, A61L 9/012, A61L 9/05, E03D 9/02, E03D 9/03

(54) **SPENDER UND DAMIT AUSGESTATTETE SANITÄREINRICHTUNG**

(30) Priorität: 23.12.2014 DE 102014119536
(62) Teilanmeldung aus: 15201186.2
(71) Anmelder: Villeroy & Boch AG, 66693 Mettlach (DE)
(72) Erfinder: Legrix, Jean-Christophe, 66111 Saarbrücken (DE)
(74) Vertreter: Kilchert, Jochen

(57) **Zusammenfassung**

Die Erfindung betrifft einen Spender (10) zur Abgabe von Reinigungs-, Desinfektions- und/oder Duftstoff (20) an eine Sanitäreinrichtung (30), wobei der Spender (10) einen Rohrabschnitt (40) aufweist, der geeignet ist in einem Wasserzulauf zu einer Sanitäreinrichtung (30) angeordnet zu werden, wobei der Rohrabschnitt (40) einen öffen- und wiederverschließbaren Zugang, insbesondere eine Zugangsöffnung (50), aufweist, durch welche ein Reinigungs-, Desinfektions- und/oder Duftstoff (20) in einen an die Zugangsöffnung angrenzenden Abschnitt (60) des Rohrabschnitts (40) eingebracht und dort so gehalten werden kann, dass der Reinigungs-, Desinfektions- und/oder Duftstoff (20) bei einem Spülvorgang zumindest teilweise umspült oder ausgespült wird, sowie eine damit ausgestattete Sanitäreinrichtung.

## Beschreibung

Die Erfindung betrifft einen Ständer zur Abgabe von Reinigungs-, Desinfektions- und/oder Duftstoff an eine Sanitäreinrichtung sowie eine mit einem solchen Spender ausgestattete Sanitäreinrichtung gemäß den Oberbegriffen der Patentansprüche 1 und 16.

Spender zur Abgabe von Reinigungs-, Desinfektions- und/oder Duftstoff an eine Sanitäreinrichtung sind aus dem Stand der Technik seit geraumer Zeit bekannt. Derartige Spender werden üblicherweise dazu verwendet, Toiletten in einem hygienischen Zustand zu halten und/oder einen Duftstoff abzugeben. Gemäß dem Stand der Technik werden die Reinigungs-, Desinfektions- und/oder Duftstoffmittel zu diesem Zweck in Abgabebehälter, wie beispielsweise korbförmige Behälter, gefüllt, die sodann so in einer Toilettenschüssel positioniert werden, dass diese bei einem Spülvorgang von Wasser umspült werden, so dass ein Teil des in dem Korb befindlichen Reinigungs-, Desinfektions- und/oder Duftstoffs aus dem Korb ausgespült wird.

Ein Nachteil der Verwendung dieser unmittelbar in eine Toilettenschüssel einhängbaren Körbe besteht darin, dass diese in Kontakt mit Urin und/oder Fäkalien kommen können, was zum einen unhygienisch ist und ferner einen Austausch der Körbe und/oder ein Nachfüllen derartiger Behälter unangenehm macht. Darüber hinaus kann ein solcher Korb, der notwendigerweise im Spülstrom des Spülwassers für die Toilettenschüssel angeordnet sein muss, damit seine Inhaltsstoffe während eines Spülvorgang gelöst und mit einem wirksamen Anteil ausgespült werden können, die Spülung der Toilettenschüssel und damit einem Austrag des wegzuspülenden Gutes beeinträchtigen. Ferner findet bei der Verwendung derartiger Körbe während eines Spülvorgangs keine gleichmäßige Verteilung des Reinigungs-, Desinfektions- und/oder Duftstoffmittels statt; vielmehr erfolgt eine solche Verteilung zumeist nur lokal beschränkt um einen Bereich um den Korb bzw. Behälter herum, so dass bestimmte Bereiche einer Toilettenschüssel nicht oder nur sehr unzureichend von dem Reinigungs-, Desinfektions- und/oder Duftstoff benetzt werden. Ein weiterer Nachteil derartiger Körbe zur Aufnahme von Reinigungs-, Desinfektions- und/oder Duftstoff besteht ferner darin, dass diese bei neuartigen randlosen Toiletten und WCs nicht funktionsfähig sind, da diese WCs keinen Spülrand besitzen und ein am Rand angebrachter Korb somit nicht mehr im Strömungsbereich des Spülwassers liegt.

Gemäß einer weiteren aus dem Stand der Technik bekannten Alternative werden derartige Reinigungs-, Desinfektions- und/oder Duftstoffe innerhalb des Wasserkastens eines Vorwandelements eingesetzt. Bei dieser Lösung kommt der Reinigungs-, Desinfektions- und/oder Duftstoff in Kontakt mit den im Wasserkasten angeordneten Komponenten, wie beispielsweise Ventilen oder Dichtungen, wobei diese Komponenten durch die eingesetzten Mittel beschädigt werden können.

Aus diesem Grund wurden in der Vergangenheit Lösungen entwickelt, bei welchen ein Reinigungs-, Desinfektions- und/oder Duftstoff nicht mittels eines Korbs in die Toilettenschüssel eingehängt oder in einem Spülwasserbehälter eingesetzt wird. Bei diesen aus dem Stand der Technik bekannten Lösungen wird eine konstruktiv speziell angepasste Sanitärschüssel, wie beispielsweise Toilettenschüssel verwendet, bei welcher im rückwärtigen, einem Spülwasserzulauf zugewandten Teil der Sanitäreinrichtung ein Hohlraum vorgesehen ist, in welchen ein Reinigungs-, Desinfektions- und/oder Duftstoff eingebracht werden kann, der während eines Spülvorgangs mit Spülwasser in Kontakt kommt und mit dem Spülwasser teilweise in die Toilettenschüssel ausgetragen wird. Derartig modifizierte Toilettenschüsseln sind beispielsweise in der AU 151 16/70 oder der US 1,178,852 beschrieben. Ein wesentlicher Nachteil dieser Lösungen besteht darin, dass die Anfertigung derartig konstruktiv angepasster Schüsseln für Sanitäreinrichtungen sehr aufwändig und damit teuer ist und darüber hinaus nur ein Einsatz von festen Reinigungs-, Desinfektions- und/oder Duftstoffmitteln in Frage kommt. Darüber hinaus erfährt der Spülstrom bei den vorgenannten modifizierten Sanitärschüsseln einen starken Druckverlust, da das Spülwasser durch die in der Keramik für den Reinigungs-, Desinfektions- und/oder Duftstoff angelegten Hohlräume strömen muss, um Reinigungs-, Desinfektions- und/oder Duftstoff wirksam lösen und ausspülen zu können, was zu einem schlechten Spülergebnis führt.

Somit sind aus dem Stand der Technik zwar Spender zur Abgabe von Reinigungs-, Desinfektions- und/oder Duftstoffmitteln an eine Sanitäreinrichtung bekannt. Diese weisen jedoch erhebliche Nachteile auf, die zum einen in einer unhygienischen Handhabbarkeit begründet liegen, da beispielsweise ein das Reinigungs-, Desinfektions- und/oder Duftstoffmittel aufweisender Korb am Rand einer Toilettenschüssel eingehängt wird und somit in Kontakt mit Fäkalien und Urin kommen kann. Darüber wird durch einen derartig eingehängten Korb auch die Optik eines WCs beeinträchtigt, da der Korb sichtlich kein Teil der Keramik oder der Toilettenschüssel oder des Urinals ist und als Fremdkörper wirkt. Ferner hindert ein derartig eingehängter Korb zum einen das Spülergebnis, wobei ferner auch eine Verteilung des Reinigungs-, Desinfektions- und/oder Duftstoffmittels nur sehr unvollständig, teilweise nur lokal, keinesfalls jedoch gleichmäßig erfolgt. Des Weiteren sind bislang bekannte Lösungen auf den Aggregatszustand des Reinigungs-, Desinfektions- und/oder Duftstoffmittels festgelegt, d.h. dass üblicherweise entweder WC-Steine oder flüssige Reinigungsmittel verwendet werden können, keinesfalls können jedoch flüssige oder feste Reinigungsmittel alternativ verwendet werden. Im Übrigen ist eine Nachfüllbarkeit derartiger Einhängkörbe unhyienisch und wird deshalb üblicherweise nur bei in eine modifizierte Sanitärkeramik integrierten Aufnahmebehältern für Reinigungs-, Desinfektions- und/oder Duftstoffe durchgeführt, was eine Entsorgung leerer Einhängkörbe notwendig macht, wodurch wiederum ein Abfallvolumen vergrößert wird. Darüber hinaus stellen in eine Toilettenschüssel eingehängte Körbe insbesondere für Kinder ein Sicherheitsrisiko dar, da sich diese Körbe in direkter Reichweite von Kindern befinden. Dies ist insbesondere deshalb problematisch, da Reinigungs-, Desinfektions- und/oder Duftstoffmittel, die zur Abgabe an eine Sanitäreinrichtung bestimmt sind, in aller Regel von Kindern ferngehalten werden sollten und einen sorgfältigen Umgang erfordern.

Der Erfindung liegt die Aufgabe zu Grunde, einen Spender zur Abgabe von Reinigungs-, Desinfektions- und/oder Duftstoffmittel an eine Sanitäreinrichtung sowie eine damit ausgestattete Sanitäreinrichtung zur Verfügung zu stellen, wobei die vorgenannten Nachteil vermieden werden und wobei der Spender auf einfache und kostengünstige Weise herstellbar sowie insbesondere nachfüllbar sein soll.

Diese Aufgabe wird durch einen Spender gemäß Patentanspruch 1 sowie durch eine Sanitäreinrichtung gemäß Patentanspruch 16 gelöst.

Insbesondere wird die Aufgabe durch einen Spender zur Abgabe von Reinigungs-, Desinfektions- und/oder Duftstoff an eine Sanitäreinrichtung gelöst, wobei der Spender einen Rohrabschnitt aufweist, der geeignet ist, in einem Wasserzulauf zu einer Sanitäreinrichtung angeordnet zu werden, wobei der Rohrabschnitt einen öffen- und wiederverschließbaren Zugang, insbesondere eine Zugangsöffnung, aufweist, durch welche ein Reinigungs-, Desinfektions- und/oder Duftstoff in einen an die Zugangsöffnung angrenzenden Abschnitt des Rohrabschnitts eingebracht und dort so gehalten werden kann, dass der Reinigungs-, Desinfektions- und/oder Duftstoff bei einem Spülvorgang zumindest teilweise umspült und teilweise, d.h. in einen vorbestimmten Maß, ausgespült wird.

Ein wesentlicher Punkt der Erfindung besteht darin, dass der Spender erfindungsgemäß als Rohrabschnitt vorliegt, der in einem Wasserzulauf zu einer Sanitäreinrichtung angeordnet werden kann. Auf diese Weise kann der erfindungsgemäße Spender mit jedem gängigen Wasserklosett (WC), Urinal oder Bidet verwendet werden, ohne dass eine Modifizierung oder eine spezielle Ausbildung der jeweiligen Sanitärschüssel notwendig wäre. Ferner kann der erfindungsgemäße Spender auf diese Weise problemlos mit üblichen Wasserklosetts mit Spülrand, jedoch auch mit neuen randlosen Wasserklosetts verwendet werden, da der erfindungsgemäße Spender nicht in die Toilettenschüssel eingehängt wird sondern unmittelbar bereits im Wasserzulauf der Sanitäreinrichtung wirkt.

Da der Spender, respektive dessen Rohrabschnitt ferner im Wasserzulauf zu der Sanitäreinrichtung angeordnet ist, ist eine Zugabe von Reinigungs-, Desinfektions- und/oder Duftstoffmittel an oder in einen Spülwasserbehälter des Wasserklosetts nicht notwendig, so dass eine Beschädigung von Komponenten in dem Spülwasserbehälter mit dem erfindungsgemäßen Spender funktionsbedingt nicht möglich ist und somit jedenfalls vermieden wird.

Zum Einbringen von Reinigungs-, Desinfektions- und/oder Duftstoffmittel weist der Rohrabschnitt des erfindungsgemäßen Spenders einen Zugang auf, der geöffnet und wiederverschlossen werden kann. Dieser Zugang, der auch als Zugangsöffnung bezeichnet werden kann, ist so ausgebildet, dass ein Reinigungs-, Desinfektions- und/oder Duftstoff so in den Rohrabschnitt eingebracht und dort gehalten werden kann, dass der Reinigungs-, Desinfektions- und/oder Duftstoff bei einem Spülvorgang zumindest teilweise umspült, oder, beispielsweise aus einer entsprechenden, in dem Rohrabschnitt angeordneten, Dosiervorrichtung ausgespült wird. Zu diesem Zweck ist der Reinigungs-, Desinfektions- und/oder Duftstoff durch die Zugangsöffnung so in den Rohrabschnitt eingesetzt, dass er in eingesetztem Zustand im Bereich von 5% bis 60%, bevorzugt im Bereich von 8% bis 55% und besonders bevorzugt im Bereich von 12,5% bis 50%, jeweils bezogen auf den Durchmesser des Rohrabschnitts in den Rohrabschnitt ragt. Unter Durchmesser des Rohrabschnitts wird in diesem Zusammenhang die lichte Weite des Rohrabschnitts, ausgehend von der Zugangsöffnung an dem Rohrabschnitt bis zur gegenüberliegenden Seite des Rohrabschnitts verstanden.

Erfindungsgemäß ist der Spender so in den Wasserzulauf zu einer Sanitäreinrichtung eingebaut, dass der Zugang, respektive die Zugangsöffnung des Rohrabschnitts im bestimmungsgemäßen Gebrauch an einer Oberseite des Rohrabschnitts angeordnet ist, wobei der Rohrabschnitt in diesem Falle im Wesentlichen horizontal verläuft, und der Reinigungs-, Desinfektions- und/oder Duftstoff von oben in den Rohrabschnitt eingebracht wird.

Alternativ kann der Rohrabschnitt des Spenders auch in einem vertikalen Rohr des Wasserzulaufs der Sanitäreinrichtung angeordnet sein, wobei sich die Zugangsöffnung in diesem Fall an einem Seitenabschnitt des Rohrabschnitts befindet, und der Reinigungs-, Desinfektions- und/oder Duftstoff seitlich in den Rohrabschnitt eingebracht wird.

Gemäß einer Ausführungsform der Erfindung ist der Zugang, respektive die Zugangsöffnung, unmittelbar an dem Rohrabschnitt angeordnet und kann von einem Deckel verschlossen werden.

Gemäß einer Alternative kann der Zugang zu dem Rohrabschnitt auch über einen Zugangsabschnitt realisiert sein, der selbst rohrförmig mit einem runden, ovalen oder eckigen Querschnitt ausgeführt ist. Der Zugangsabschnitt erstreckt sich vorzugsweise winkelig, insbesondere rechtwinklig, von dem Rohrabschnitt weg.

Der Zugangsabschnitt ist gemäß dieser Ausführungsform der Erfindung an dem Rohrabschnitt befestigt, wobei insbesondere eine Steck- oder Schraubverbindung bevorzugt ist, bei welcher der Zugangsabschnitt in eine Zugangsöffnung des Rohrabschnitts eingesteckt, oder bevorzugt eingeschraubt ist. Auf diese Weise ist es beispielsweise möglich, unterschiedliche Zugangsabschnitte zu verwenden, so dass der erfindungsgemäße Spender beispielsweise unterschiedlich lang ausgebildet sein und an verschiedene Toilettenformen angepasst werden kann.

Im Falle der Verwendung eines Zugangsabschnitt, der nicht einstückig mit dem Rohrabschnitt, sondern als separates Teil vorliegt und mit dem Rohrabschnitt verbunden werden kann, kann der Rohrabschnitt beispielsweise in einem hinteren Bereich oder an einer, vorzugsweise nicht sichtbaren, Stelle an der Unterseite der Keramik der Sanitäreinrichtung im Wasserzulauf zu der Sanitäreinrichtung angeordnet sein, während der Zugangsabschnitt durch eine in der Keramik vorgesehene Bohrung geführt wird und durch diese Bohrung hindurch mit dem Rohrabschnitt verbunden wird. Auf diese Weise kann der erfindungsgemäße Spender so in eine Sanitäreinrichtung eingebaut werden, dass an der Oberseite der Keramik der Sanitäreinrichtung nur ein Deckel des Spenders sichtbar ist, während sich der Zugangsabschnitt innerhalb der Keramik bis zu dem Rohrabschnitt erstreckt, der seinerseits im Inneren der Keramik oder unterhalb einer oberen Keramikblende verläuft und auf diese Weise für einen Benutzer der Sanitäreinrichtung quasi unsichtbar ist.

Alternativ kann der Zugangsabschnitt auch einstückig mit dem Rohrabschnitt ausgebildet sein. In diesem Fall ist der Rohrabschnitt in einem hinteren Bereich der Sanitäreinrichtung in den Wasserzulauf der Sanitäreinrichtung eingebaut, während sich der Zugangsabschnitt ebenfalls hinter der Sanitäreinrichtung, respektive der Toilettenschüssel befindet.

Ein Nachfüllen des erfindungsgemäßen Spenders erfolgt durch ein Abnehmen des Deckels des Spenders und ein Ein-, respektive Nachfüllen von Reinigungs-, Desinfektions- und/oder Duftstoff sowie ein Wiederverschließen des Deckels.

Gemäß einer Ausführungsform der Erfindung kann das Reinigungs-, Desinfektions- und/oder Duftstoffmittel fest, gelartig und/oder flüssig sein, wobei zur Aufnahme des Reinigungs-, Desinfektions- und/oder Duftstoffmittels vorzugsweise ein für Wasser zumindest teildurchlässiger Behälter, insbesondere mit einer Membran, ein Korb oder ein Sieb, vorgesehen ist, der in den Zugang und/oder den Zugangsabschnitt einsteck- oder einhängbar ist.

Des Weiteren kann der Spender eine durch Luft- und/oder Wasserdruck betätigbare Dosiereinrichtung zur Abgabe von gelartigem und/oder flüssigem Reinigungs-, Desinfektions- und/oder Duftstoff aufweisen.

Die Dosiereinrichtung weist erfindungsgemäß ein Ventil und/oder eine durch eine Vorspannung geschlossen gehaltene Verschlussklappe auf, wobei das Ventil und/oder die Verschlussklappe während eines Spülvorgangs durch einen hierbei entstehenden Luft- und/oder Wasserdruck temporär geöffnet wird/werden und sich das Ventil und/oder die Verschlussklappe bei einem nachlassenden Luft- und/oder Wasserdruck automatisch wieder schließt/schließen.

Auf diese Weise ist es möglich, den Spender alternativ und nach Wunsch mit einem festen, gelartigen oder flüssigen Reinigungs-, Desinfektions- und/oder Duftstoff zu verwenden, wobei ein entsprechender, für Wasser zumindest teildurchlässiger Behälter verwendet werden kann, so dass bei einem Spülvorgang eine vordefinierbare Menge an Reinigungs-, Desinfektions- und/oder Duftstoff abgegeben werden kann.

In diesem Zusammenhang sei darauf hingewiesen, dass der Zugangsabschnitt erfindungsgemäß so gestaltet sein kann, dass dieser einen, zwei oder drei parallel verlaufende Zugänge zu dem Rohrabschnitt aufweist, wobei in jedem dieser Zugänge, jedoch voneinander getrennt, ein Reinigungs-, Desinfektions- und/oder Duftstoff eingefüllt werden kann, so dass nach Wunsch unterschiedliche Reinigungs-, Desinfektions- und/oder Duftstoffmittel verwendet werden können. Alternativ kann auch der zur Aufnahme der Reinigungs-, Desinfektions- und/oder Duftstoffmittel vorgesehene Behälter mehrere Abteile aufweisen, die jeweils separat zur Aufnahme eines Reinigungs-, Desinfektions- und/oder Duftstoffmittels vorgesehen sind. Auf diese Weise ist es beispielsweise auch möglich, nur ein Reinigungsmittel, oder nur ein Desinfektionsmittel oder nur ein Duftstoffmittel oder eine Kombination von jeweils zweien der vorgenannten Stoffe zu verwenden. Sowohl der Zugangsabschnitt als auch der Behälter sind hierbei so gestaltet, dass die Abteile für die unterschiedlichen Stoffe in eingesetztem Zustand in den Rohrabschnitt ragen und bei einem Spülvorgang jeweils von Spülwasser umspült werden.

Die Verwendung einer einstellbaren Dosiereinrichtung bietet den weiteren Vorteil, dass die Menge an abzugebendem Reinigungs-, Desinfektions- und/oder Duftstoffmittel eingestellt werden kann. Insbesondere ist durch die Verwendung einer Dosiereinrichtung der Einsatz von gelartigen oder flüssigen Reinigungs-, Desinfektions- und/oder Duftstoffmitteln auf einfache Weise möglich.

Für den Fall, dass ein festes Reinigungs-, Desinfektions- und/oder Duftstoffmittel verwendet wird, so kann dieses blockartig ausgebildet sein. Eine solche blockartige feste Ausgestaltung des Reinigungs-, Desinfektions- und/oder Duftstoffmittels bietet den Vorteil, dass dieser Block mit einer Halte- oder Klemmvorrichtung zum unmittelbaren Einsatz in den Zugang und/oder den Zugangsabschnitt versehen sein kann. Auf diese Weise kann auf einen Aufnahmebehälter für das Reinigungs-, Desinfektions- und/oder Duftstoffmittel verzichtet werden.

Des Weiteren ist der Zugang oder Zugangsabschnitt erfindungsgemäß mit einem Deckel dicht, insbesondere kindersicher, verschließbar, insbesondere abschließbar.

Gemäß einer weiteren Ausführungsform der Erfindung weist der Rohrabschnitt an seinem/seinen Ende(n) ein Gewinde und/oder einen Steckanschluss auf, das bzw. der mit einem Dichtungsmittel, ggf. mit Lamellen, versehen ist. Mittels eines solchen Schraub- oder Steckanschlusses kann der Rohrabschnitt erfindungsgemäß in eine, vorteilhafterweise genormte, Spülwasserzulauföffnung der Sanitäreinrichtung, beispielsweise einer Toilettenschüssel, eines Urinals oder eines Bidets eingesteckt oder eingeschraubt werden, während das andere Ende des Rohrabschnitts mit einer Wasserversorgung verbindbar ist.

Gemäß einer weiteren vorteilhaften Ausführungsform der Erfindung weist der Zugang und/oder der Zugangsabschnitt ein Rückhalte- bzw. Rückschlagventil, beispielsweise in Form von selbstschließenden Gummilippen auf, welche bei einem Spülvorgang mit geöffneter Zugangsöffnung einen Wasseraustritt aus der Zugangsöffnung verhindern. Diese Ausführungsform stellt ein Sicherheitssystem dar, das verhindert, dass selbst bei geöffneter Zugangsöffnung ein versehentlich eingeleiteter Spülvorgang im Wesentlichen nicht zu einem ungewollten Wasseraustritt aus der Zugangsöffnung führt, da dies durch das Rückhalteventil, beispielsweise entsprechende Dichtlippen oder einen Schwimmer, verhindert wird. Gemäß einer weiteren Ausführungsform der Erfindung ist der Rohrabschnitt im Bereich des Zugangs in den Rohrabschnitt hinein verbreitert ausgebildet. Eine solche Ausführungsform ermöglicht in vorteilhafter Weise eine im Wesentlichen druckverlustfreie Umspülung des Reinigungs-, Desinfektions- und/oder Duftstoffmittels, oder respektive dessen Behälters. Darüber hinaus kann ein solcher Behälter oder ein festes, blockartig ausgebildetes Reinigungs-, Desinfektions- und/oder Duftstoffmittel auch in Richtung einer Strömungsrichtung stromlinienförmig ausgebildet sein, so dass der Behälter oder der eingehängte Block während eines Spülvorgangs im Wesentlichen druckverlustfrei umspült wird.

Des Weiteren wird die erfindungsgemäße Aufgabe insbesondere auch durch eine Sanitäreinrichtung, insbesondere eine Toilette, ein Urinal oder ein Bidet gelöst, das mit einem Spender gemäß vorstehenden Ausführungen ausgestattet ist.

Erfindungsgemäß ist der erfindungsgemäße Spender aus Kunststoff hergestellt, wobei darauf hingewiesen sei, dass auch andere Materialien, wie Metall oder Keramik verwendet werden können.

Zusammenfassend kann der Gegenstand der Erfindung, der, als Spender ausgeführt, im Wesentlichen einen Adapter darstellt, der in eine Wasserzulaufleitung zu einer Sanitäreinrichtung integriert werden kann und zur Abgabe von Reinigungs-, Desinfektions- und/oder Duftstoffmitteln an eine Sanitäreinrichtung geeignet ist, wie folgt beschrieben werden.. Der Adapter ist in Form eines Spenders gemäß vorstehenden Ausführungen ausgebildet und bildet eine Schnittstelle zwischen einem Wasserzulauf und einer Sanitäreinrichtung, wie beispielsweise einem WC, Urinal oder Bidet, wobei der Adapter im Falle eines Bidets vorzugsweise an oder in die Wasserzulaufleitung integriert ist, die, anders als im Falle eines Urinals oder WCs, nicht ein Spülfluid, sondern eine Waschflüssigkeit in das Bidet leitet.

In vorteilhafter Weise ist die Erfindung insbesondere für ein wandhängendes WC oder ein WC geeignet, das nahe an einer Wand steht, da der erfindungsgemäße Spender in diesem Fall sehr einfach mit einer Spülwasserzulauföffnung des WCs verbunden werden kann, die vorteilhafterweise gemäß der Norm EN997 definiert ist.

Für den Fall eines erfindungsgemäßen Spenders, der aus einem Rohrabschnitt mit einem daran anbringbaren Zugangsabschnitt besteht, besitzt das WC eine in der Keramik der Toilettenschüssel ausgebildete Bohrung, durch welches der Zugangsabschnitt hindurchgeführt werden kann, um mit dem Rohrabschnitt verbunden zu werden.

Sowohl der Zugangsabschnitt als auch die sich durch die WC-Keramik hindurcherstreckende Bohrung sind hinsichtlich ihrer Geometrie so aufeinander abgestimmt, dass der Zugangsabschnitt einfach durch diese Bohrung hindurchgeführt werden kann, wobei sowohl der Zugangsabschnitt als auch die Bohrung so groß gestaltet sind, dass, mit Blick auf möglichst lange Wartungs-, d.h. Nachfüllintervalle, eine möglichst große Menge an Reinigungs-, Desinfektions- und/oder Duftstoffmittel in den Zugangsabschnitt eingefügt werden kann. Zu diesem Zweck ist die lichte Breite sowohl der Bohrung als auch des Zugangsabschnitts so bemessen, dass auch große WC-Steine in den Zugangsabschnitt eingefügt werden können. Besonders im Falle eines Zugangsabschnitts, der mehrere Abteile für Reinigungsmittel, Desinfektionsmittel und/oder Duftstoff umfasst oder einen Behälter mit mehreren Abteilen aufnehmen kann, hat es sich als vorteilhaft erwiesen, wenn sowohl Bohrung als auch Zugangsabschnitt einen Minimaldurchmesser von 3 cm, bevorzugt 4 cm und besonders bevorzugt mehr als 4 cm aufweisen. In diesem Zusammenhang sei erwähnt, dass der Zugangsabschnitt vorzugsweise röhrenförmig mit einem einheitlichen runden Durchmesser ausgebildet ist.

Da der das Reinigungs-, Desinfektions- und/oder Duftstoffmittel aufweisende Behälter erfindungsgemäß einfach in den Zugangsabschnitt einhängbar ist, kann dieser Behälter zum Nachfüllen oder Austauschen einfach aus dem Zugangsabschnitt entnommen werden. Im Anschluss ist es möglich, entweder einen wiederaufgefüllten Behälter oder einen neuen Behälter mit frischem Reinigungs-, Desinfektions- und/oder Duftstoffmittel in den Zugangsabschnitt oder alternativ einen festen Reinigungs-, Desinfektions- und/oder Duftstoffblock einzusetzen. Der Behälter oder das feste blockförmige Reinigungs-, Desinfektions- und/oder Duftstoffmittel wird so weit in den Rohrabschnitt eingeführt, dass er gemäß einer Ausführungsform zu etwa einem Achtel bis etwa zur Hälfte über die lichte Weite des Spülrohres ragt und auf diese Weise während eines Spülvorgangs von Spülwasser umspült wird, ohne dass der Spülstrom spürbar gebremst wird.

Im Falle der Verwendung eines korb- oder siebartigen Behälters zum Einsatz mit einem WC-Stein ist der Korb, Beutel oder das Sieb zu etwa 30% bis 70% für Wasser und das Reinigungs-, Desinfektions- und/oder Duftstoffmittel durchlässig, beispielsweise derart, dass 30% bis 70% seiner Fläche mit Durchlässen versehen sind, durch welche Wasser und Reinigungs-, Desinfektions- und/oder Duftstoffmittel strömen kann.

Im Falle des Einsatzes von flüssigem Reinigungs-, Desinfektions- und/oder Duftstoff kann der Behälter als Reservoir ausgebildet sein, auf welches während eines Spülvorgangs ein Luft- und/oder Wasserdruck wirkt, der dazu führt, dass Reinigungs-, Desinfektions- und/oder Duftstoffmittel aus dem Reservoir austritt.

Sofern der erfindungsgemäße Spender mit einem Deckel verschlossen ist, stellt dieser gegebenenfalls auch einen Verschluss für eine in der Keramik vorgesehene Bohrung dar, durch welche sich gemäß einer Ausführungsform der Zugangsabschnitt erstreckt. Der Deckel ist nach Wunsch vandalismus- und kindersicher verschließbar und/oder abschließbar.

Weitere Ausführungsformen der Erfindung ergeben sich aus den Unteransprüchen.

Nachfolgend wird die Erfindung anhand eines Ausführungsbeispiels beschrieben, das anhand der Abbildungen näher erläutert wird. Hierbei zeigen:
- Fig. 1: eine schematische Darstellung eines erfindungsgemäßen Spenders gemäß einer Ausführungsform der Erfindung mit abnehmbarem Zugangsabschnitt in Einzelteilansicht;
- Fig. 2: eine schematische Darstellung eines erfindungsgemäßen Spenders gemäß der in Fig. 1 dargestellten Ausführungsform in zusammengebauter Schnittansicht;
- Fig. 3: eine schematische Darstellung eines erfindungsgemäßen Spenders gemäß der in Fig. 1 dargestellten Ausführungsform in zusammengebauter Außenansicht;
- Fig. 4: eine schematische Darstellung des erfindungsgemäßen Spenders im in eine Sanitäreinrichtung eingebauten Zustand in perspektivischer Schnittansicht;
- Fig. 5: die Ausführungsform gemäß Fig. 1 in schematischer Darstellung, wobei der Rohrabschnitt in eine Sanitäreinrichtung eingesetzt ist; und
- Fig. 6: die Ausführungsform gemäß Fig. 5, wobei der Spender in der Sanitäreinrichtung zusammengesetzt und verschlossen ist.

In der nachfolgenden Beschreibung werden für gleiche und gleich wirkende Teile dieselben Bezugsziffern verwendet.

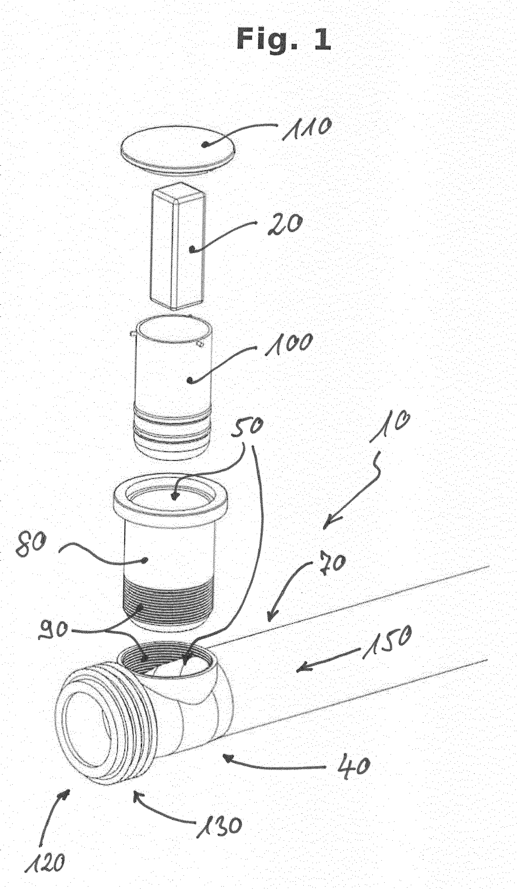

Fig. 1 zeigt einen erfindungsgemäßen Spender 10, bestehend aus einem Rohrabschnitt 40, der eine Zugangsöffnung 50 aufweist, die mit einem Gewinde versehen ist, in welche ein Zugangsabschnitt 80, der rohrabschnittseitig ebenfalls ein Gewinde aufweist, eingeschraubt werden kann.

Über diese Schraubverbindung 90 lässt sich der Zugangsabschnitt 80 mit dem Rohrabschnitt 40 dicht verbinden, so dass der Zugangsabschnitt 80 in Kommunikation mit dem Rohrabschnitt 40 ist.

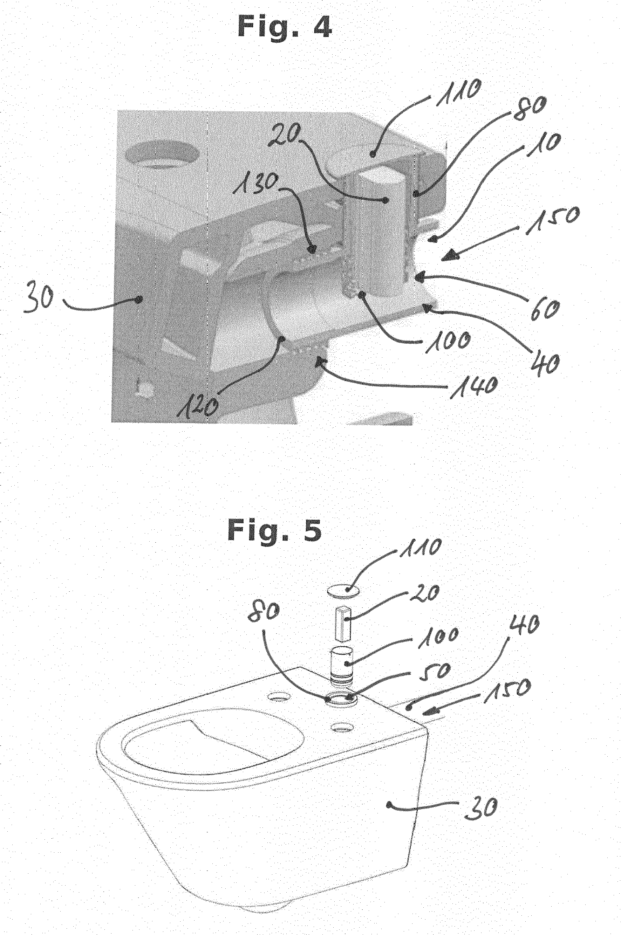

Der Rohrabschnitt 40 weist ferner ein Ende 120 auf, das zum Einsatz in eine Spülwasserzulauföffnung 140, die in Fig. 4 dargestellt ist, geeignet ist. Das Ende 120 des Rohrabschnitts 40 ist zu diesem Zweck mit Lamellen 130 versehen.

Des Weiteren weist der erfindungsgemäße Spender einen Behälter 100 auf, der mit einem Reinigungs-, Desinfektions- und/oder Duftstoffmittel 20 bestückt werden kann. Der Behälter 100 wird nach seiner Befüllung oder leer, um anschließend befüllt zu werden, in den Zugangsabschnitt 80 eingesetzt, wobei der Behälter 100 in eingesetztem Zustand in die lichte Weite des Rohrabschnitts 40 ragt und sich zu etwa einem Drittel über die lichte Weite des Rohrabschnitts 40 erstreckt.

In seinem in die lichte Weite des Rohrabschnitts ragenden Teil ist der Behälter 100 wasserdurchlässig gestaltet, so dass in Strömungsrichtung 150 fließendes Spülwasser den Behälter 100 und das darin befindliche Reinigungs-, Desinfektions- und/oder Duftstoffmittel 20 umspült und zu einem vordefinierten Anteil zusammen mit dem Spülwasser aus dem Ende 120 in die Spülwasserzulauföffnung 140 der Sanitäreinrichtung 30 spült. Die Zugangsöffnung 50 kann mit einem Deckel 110 verschlossen werden.

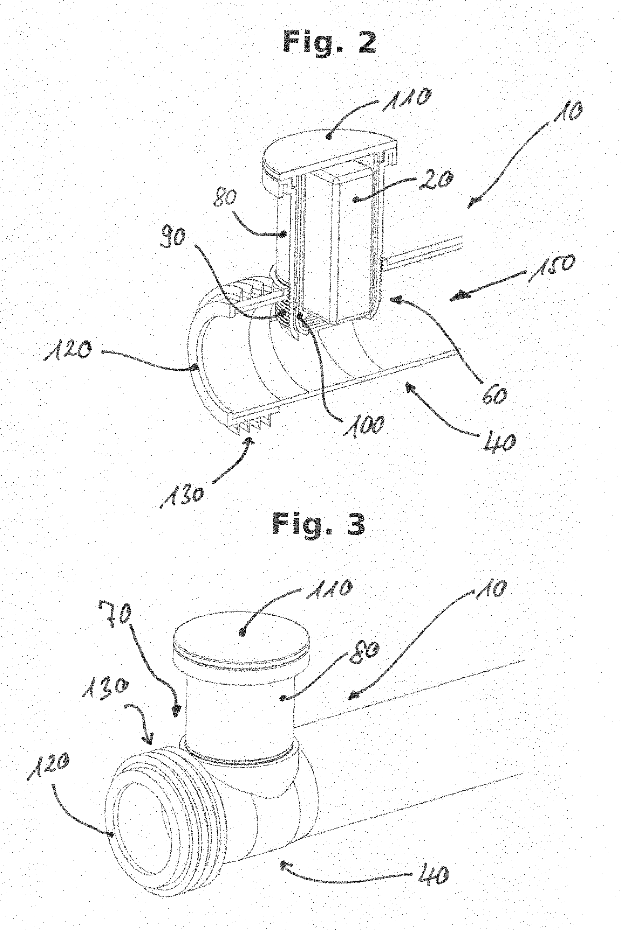

Fig. 2 zeigt in schematischer Darstellung die Komponenten gemäß Fig. 1 in zusammengebauter Darstellung mit aufgesetztem Deckel 110.

Der Zugangsabschnitt 80 ist hierbei in die mit einer Schraubverbindung 90 versehene Zugangsöffnung 50 an der Oberseite 70 des Rohrabschnitts 40 eingeschraubt.

Wie in Fig. 2 zu erkennen ist, erstreckt sich der Behälter 100 über ca. ein Drittel der lichten Weite des Rohrabschnitts 40, wobei der Gewindeabschnitt der Schraubverbindung 90 des Zugangsabschnitts 80 in seinem unteren Teil, ebenso wie der Behälter 100, wasserdurchlässig ausgebildet ist, d.h. mit Durchgangsöffnungen versehen ist. Gemäß einer weiteren, alternativen Ausführungsform der Erfindung ist der Behälter 100 länger ausgebildet als der Zugangsabschnitt 80, so dass der Behälter 100 im Bereich des Rohrabschnitts 40 aus dem Zugangsabschnitt 80 herausragt. In jedem Fall kann wird das in dem Behälter 100 befindliche Reinigungs-, Desinfektions- und/oder Duftstoffmittel 20 während eines Spülvorgangs von Spülwasser umspült und in einem vorgestimmten Maß aus dem Behälter 100 ausgetragen.

Fig. 3 zeigt den erfindungsgemäßen Spender gemäß Fig. 2 in geschlossener Darstellung.

Fig. 4 zeigt die schematische Darstellung eines erfindungsgemäßen Spenders 10, der in eine Spülwasserzulauföffnung 140 einer Sanitäreinrichtung 30 eingesetzt ist. Gemäß Fig. 4 ist gut zu erkennen, dass der Behälter 100, in welchem sich das Reinigungs-, Desinfektions- und/oder Duftstoffmittel 20 befindet, mit Öffnungen versehen ist, so dass Wasser in Strömungsrichtung 150 durch diesen Behälter 100 strömen kann, um Reinigungs-, Desinfektions- und/oder Duftstoffmittel 20 auszuspülen und durch das Ende 120 des Rohrabschnitts 40 in die Sanitäreinrichtung 30 zu leiten.

In Fig. 5 ist eine Sanitäreinrichtung 30 dargestellt, in die in einem hinteren, einer Spülwasserzuleitung zugewandten Teil ein Rohrabschnitt 40 eines erfindungsgemäßen Spenders eingesetzt ist. Durch eine Bohrung in einem hinteren oberen Teil der Keramik der Sanitäreinrichtung 30 ist der Zugangsabschnitt 80 eingeführt und mit dem Rohrabschnitt 40 verschraubt. Ein Behälter 100 mit einem in diesem anzuordnenden Reinigungs-, Desinfektions- und/oder Duftstoffmittel 20 ist zum Einsatz in dem Zugangsabschnitt 80 vorgesehen. Des Weiteren kann die Zugangsöffnung 50 nach einem Einsetzen des Behälters 100 mit einem Deckel 110 verschlossen werden.

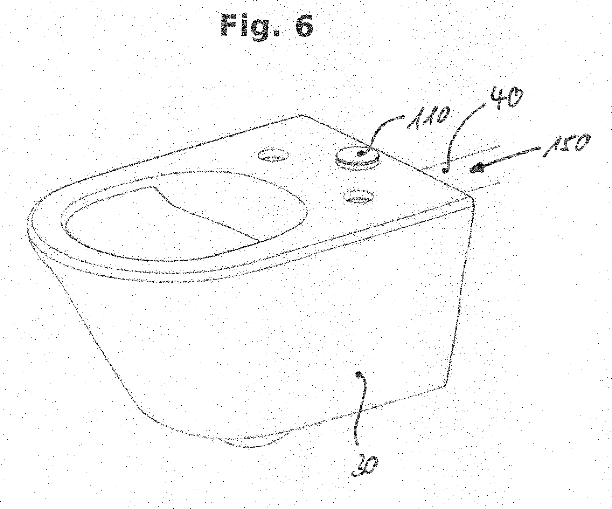

Fig. 6 zeigt den mit dem Deckel 110 verschlossenen Spender 10.

An dieser Stelle sei darauf hingewiesen, dass alle oben beschriebenen Teile für sich alleine gesehen und in jeder Kombination, insbesondere die in den Zeichnungen dargestellten Detail als erfindungswesentlich beansprucht werden. Abänderungen hiervon sind dem Fachmann geläufig.

### Bezugszeichenliste

- 10: Spender
- 20: Reinigungs-, Desinfektions- und/oder Duftstoff
- 30: Sanitäreinrichtung
- 40: Rohrabschnitt
- 50: Zugang(söffnung)
- 60: an die Zugangsöffnung angrenzender Abschnitt
- 70: Oberseite
- 80: Zugangsabschnitt
- 90: Steck- oder Schraubverbindung
- 100: Behälter
- 110: Deckel
- 120: Ende(n)
- 130: Lamellen
- 140: Spülwasserzulauföffnung
- 150: Strömungsrichtung

## Patentansprüche

1. Spender (10) zur Abgabe von Reinigungs-, Desinfektions- und/oder Duftstoff (20) an eine Sanitäreinrichtung (30), wobei der Spender (10) einen Rohrabschnitt (40) aufweist, der geeignet ist in einem Wasserzulauf zu einer Sanitäreinrichtung (30) angeordnet zu werden, wobei der Rohrabschnitt (40) einen öffen- und wiederverschließbaren Zugang, insbesondere eine Zugangsöffnung (50), aufweist, durch welche ein Reinigungs-, Desinfektions- und/oder Duftstoff (20) in einen an die Zugangsöffnung angrenzenden Abschnitt (60) des Rohrabschnitts (40) eingebracht und dort so gehalten werden kann, dass der Reinigungs-, Desinfektions- und/oder Duftstoff (20) bei einem Spülvorgang zumindest teilweise umspült oder ausgespült wird,
**dadurch gekennzeichnet, dass**
der Rohrabschnitt (40) mit einem Ende (120) in eine Spülwasserzulauföffnung (140) der Sanitäreinrichtung (30), beispielsweise einer Toilettenschüssel, eines Urinals oder eines Bidets einsteck- oder einschraubbar und mit dem anderen Ende mit einer Wasserversorgung verbindbar ist.

2. Spender nach Anspruch 1,
**dadurch gekennzeichnet, dass**
der Zugang (50) im bestimmungsgemäßen Gebrauch an einer Oberseite (70) des Rohrabschnitts (40) angeordnet ist.

3. Spender nach einem der vorhergehenden Ansprüche,
**dadurch gekennzeichnet, dass**
der Reinigungs-, Desinfektions- und/oder Duftstoff (20) in, in den Rohrabschnitt (40) eingesetzten Zustand, im Bereich von 5% bis 60%, bevorzugt im Bereich von 8% bis 55% und besonders bevorzugt im Bereich von 12,5% bis 50%, bezogen auf den Durchmesser des Rohrabschnitts (40) in den Rohrabschnitt (40) ragt.

4. Spender nach einem der vorhergehenden Ansprüche,
**dadurch gekennzeichnet, dass**
der Zugang (50) unmittelbar an dem Rohrabschnitt (40) angeordnet ist oder über einen Zugangsabschnitt (80) realisiert ist, der sich, vorzugsweise winkelig, insbesondere rechtwinkelig, von dem Rohrabschnitt (40) erstreckt.

5. Spender nach Anspruch 4,
**dadurch gekennzeichnet, dass**
der Zugangsabschnitt (50) an dem Rohrabschnitt (40) befestigt, insbesondere mittels einer Steck- oder Schraubverbindung (90) mit dem Rohrabschnitt (40) verbunden ist oder werden kann.

6. Spender nach einem der vorhergehenden Ansprüche 4 oder 5,
**dadurch gekennzeichnet, dass**
der Zugangsabschnitt (80) und der Rohrabschnitt (40) einstückig ausgebildet sind.

7. Spender nach einem der vorhergehenden Ansprüche,
**dadurch gekennzeichnet, dass**
der Reinigungs-, Desinfektions- und/oder Duftstoff (20) fest, gelartig und/oder flüssig sein kann, wobei zur Aufnahme des Reinigungs-, Desinfektions- und/oder Duftstoffs (20) ein für Wasser zumindest teildurchlässiger Behälter (100), insbesondere eine Membran, ein Korb oder ein Sieb, vorgesehen ist, der in den Zugang (50) und/oder den Zugangsabschnitt (80) einsteck- oder einhängbar ist.

8. Spender nach einem der vorhergehenden Ansprüche,
**dadurch gekennzeichnet, dass**
der Spender eine durch Luft- und/oder Wasserdruck betätigbare Dosiereinrichtung zur Abgabe von gelartigem und/oder flüssigem Reinigungs-, Desinfektions- und/oder Duftstoff (20) aufweist.

9. Spender nach Anspruch 8,
**dadurch gekennzeichnet, dass**
die Dosiereinrichtung ein Ventil und/oder eine durch eine Vorspannung geschlossen gehaltene Verschlussklappe aufweist, wobei das Ventil und/oder die Verschlussklappe während eines Spülvorgangs durch einen hierbei entstehenden Luft- und/oder Wasserdruck temporär geöffnet werden und sich das Ventil und/oder die Verschlussklappe bei einem nachlassenden Luft- und/oder Wasserdruck automatisch wieder schliessen.

10. Spender nach einem der vorhergehenden Ansprüche 1 bis 7,
**dadurch gekennzeichnet, dass**
ein fester Reinigungs-, Desinfektions- und/oder Duftstoff (20) blockartig ausgebildet und mit einer Halte- oder Klemmvorrichtung zum unmittelbaren Einsatz in den Zugang (50) und/oder den Zugangsabschnitt (80) versehen ist.

11. Spender nach einem der vorhergehenden Ansprüche,
**dadurch gekennzeichnet, dass**
der Zugang (50) oder Zugangsabschnitt (80) mit einem Deckel (110) dicht, insbesondere kindersicher, verschließbar ist.

12. Spender nach einem der vorhergehenden Ansprüche,
**dadurch gekennzeichnet, dass**
der Rohrabschnitt (40) an seinem/seinen Ende(n) (120) ein Gewinde und/oder einen Steckanschluss, gegebenenfalls mit Lamellen (130) aufweist.

13. Spender nach einem der vorhergehenden Ansprüche,
**dadurch gekennzeichnet, dass**
in dem Zugang und/oder Zugangsabschnitt (50, 80) ein Rückhalteventil, beispielsweise in Form von selbstschließenden Gummilippen vorgesehen ist, welche bei einem Spülvorgang mit geöffneter Zugangsöffnung (50) einen Wasseraustritt aus der Zugangsöffnung (50) verhindern.

14. Spender nach einem der vorhergehenden Ansprüche,
**dadurch gekennzeichnet, dass**
der Rohrabschnitt (40) im Bereich des Zugangs (50) verbreitert ist.

15. Spender nach einem der vorhergehenden Ansprüche 7 bis 14,
**dadurch gekennzeichnet, dass**
ein Behälter (100) oder ein blockartig ausgebildeter Reinigungs-, Desinfektions- und/oder Duftstoff (20) in Richtung einer Strömungsrichtung (150) während eines Spülvorgangs stromlinienförmig ausgebildet ist.

16. Sanitäreinrichtung (30), insbesondere Toilette, Urinal oder Bidet, mit einem Spender (10) gemäß einem der vorhergehenden Ansprüche.
